# DEMANDE DE BREVET EUROPEEN

(11) **EP 1 512 684 A1**
(43) Date de publication de la demande: **09.03.2005**
(21) Numéro de dépôt: 04291969.6
(22) Date de dépôt: 02.08.2004
(51) Int. Cl.: C07D 233/12, C07D 295/12, C07D 223/08

(54) **Utilisation, pour la teinture des fibres kératiniques, d'un dérivés de para-phénylènediamine substituée par un noyau homopiperidine**

(30) Priorité: 04.09.2003 FR 0310475
(71) Demandeur: L'OREAL, 75008 Paris (FR)
(72) Inventeur: Sabelle, Stéphane, 75005 Paris (FR); Ly-Carry, Thi-My, 94100 St Maur (FR)
(74) Mandataire: Fevrier, Murielle

(57) **Abrégé**

L'invention a pour objet l'utilisation, pour la teinture des fibres kératiniques, et en particulier des fibres kératiniques humaines telles que les cheveux, d'un dérivé de para-phénylènediamine substituée par un noyau homopipéridine, ainsi que le procédé de teinture mettant en oeuvre une composition comprenant au moins un tel composé.

La présente invention permet en particulier d'obtenir une coloration des fibres kératiniques puissante, peu sélective et tenace.

## Description

L'invention a pour objet l'utilisation, pour la teinture des fibres kératiniques, et en particulier des fibres kératiniques humaines telles que les cheveux, d'un dérivé de para-phénylènediamine substituée par un noyau homopipéridine.

II est connu de teindre les fibres kératiniques et en particulier les fibres kératiniques humaines telles que les cheveux avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants.

II est connu de faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4-triazole, des dérivés de pyrazolo[3,2-c]-1,2,4-triazole, des dérivés de pyrazolo[1,5-a]pyrimidine, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la para-phénylènediamine et la para-toluènediamine sont des bases d'oxydation largement utilisées. Elles permettent d'obtenir avec des coupleurs d'oxydation des nuances variées.

Cependant, il existe un besoin de découvrir de nouvelles bases d'oxydation présentant un meilleur profil toxicologique que la para-phénylènediamine et la para-toluènediamine, tout en permettant de conférer aux cheveux d'excellentes propriétés d'intensité de couleur, de variété de nuances, d'uniformité de la couleur et de la ténacité aux agents extérieurs.

Il a déjà été proposé d'utiliser des dérivés de para-phénylènediamine substituée par un noyau homopipéridine pour la coloration d'oxydation des fibres kératiniques, par exemple dans les demandes de brevet FR 2 805 737 à FR 2 805 741 et dans la demande de brevet WO 98/01106.

Le but de la présente demande est de fournir d'autres dérivés de para-phénylènediamine pour une utilisation à titre de bases d'oxydation en coloration des fibres kératiniques. En particulier, le but de la présente invention est de fournir des dérivés de para-phénylènediamine qui permettent d'obtenir une coloration des fibres kératiniques puissante, peu sélective et tenace.

Ce but est atteint avec la présente invention qui a pour objet l'utilisation, pour la teinture des fibres kératiniques, d'un dérivé de para-phénylènediamine substituée par un noyau homopipéridine de formule (I) et /
ou de l'un de ses sels d'addition : 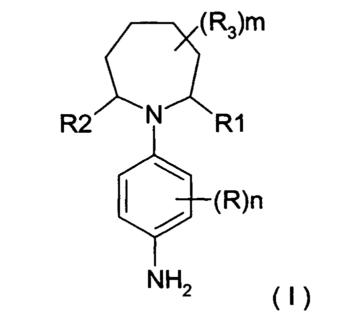 dans laquelle :
- R représente :
   - un radical alkyle ;
   - un radical hydroxyalkyle ;
   - un radical aminoalkyle ;
   - un radical hydroxy et aminoalkyle ;
   - un radical alcoxy ;
   - un radical alcoxyalkyle ;
   - un radical hydroxyalcoxy ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical carboxyalkyle ;
   - un radical carbamoylalkyle ;
- n est compris entre 0 et 4, étant entendu que lorsque n est supérieur à 1 alors les radicaux R peuvent être identiques ou différents ;
- R₁ et R₂ représentent :
   - un atome d'hydrogène ;
   - un radical alkyle saturé ou insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical hydroxyaminoalkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono ou dialkylaminocarbonyle ;
- R₃ représente :
   - un radical alkyle saturé ou insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical hydroxyaminoalkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono ou dialkylaminocarbonyle ;
   - un radical hydroxyle ;
   - un radical alcoxy ;
   - un radical hydroxyalcoxy ;
   - un radical amino ;
   - un radical mono ou dialkylamino ;
   - un radical mono ou dihydroxyalkylamino ;
   - un radical mésylate ;
   - un radical méthylsulfonamide ;
   - un radical tosylate ;
   - un radical benzènesulfonamide ;
- m est compris entre 0 et 8, étant entendu que lorsque m est supérieur à 1 alors les radicaux R₃ peuvent être identiques ou différents.

La présente invention permet en particulier d'obtenir une coloration des fibres kératiniques puissante, peu sélective et tenace.

La présente invention a également pour objet un procédé de teinture des fibres kératiniques mettant en oeuvre les dérivés de para-phénylènediamine de formule (I).

L'invention a aussi pour objet de nouvelles compositions pour la teinture des fibres kératiniques, et en particulier des fibres kératiniques humaines telles que les cheveux, comprenant, dans un milieu de teinture approprié, au moins un dérivé de para-phénylènediamine substituée par un noyau homopipéridine.

L'invention a également pour objet de nouveaux dérivés de para-phénylènediamine substituée par un noyau homopipéridine.

L'invention a enfin pour objet les dérivés de para-nitroaniline substituée par un noyau homopipéridine permettant de synthétiser les dérivés de para-phénylènediamine tels que définis précédemment.

Dans les définitions ci-dessus, les radicaux alkyle sont linéaires ou ramifiés et comprennent, sauf autre indication, de 1 à 10 atomes de carbone, de préférence de 1 à 6 atomes de carbone. Un radical alcoxy est un radical alkyl-O, le radical alkyle étant tel que défini précédemment.

On entend par radical alkyle insaturé, un alkyle de 2 à 10 atomes de carbone et comprenant une ou plusieurs doubles et / ou triples liaisons.

Un radical alkyle substitué est un alkyle mono ou polysubstitué. Par exemple, un hydroxyalkyle ou un aminoalkyle est un alkyle qui peut être substitué par un ou plusieurs groupes hydroxy ou amino.

Dans la formule (I), le radical R est par exemple choisi parmi un radical méthyle ; un radical éthyle ; un radical isopropyle ; un radical méthoxyméthyle ; un radical hydroxyméthyle ; un radical 1-carboxyméthyle ; un radical 1-aminométhyle ; un radical 2-carboxyéthyle ; un radical 2-hydroxyéthyle ; un radical 3-hydroxypropyle ; un radical 1,2-dihydroxyéthyle ; un radical 1-hydroxy-2-aminoéthyle ; un radical 1-amino-2-hydroxyéthyle ; un radical 1,2-diaminoéthyle ; un radical méthoxy ; un radical éthoxy.

Selon un mode de réalisation particulier de l'invention, n est égal à 0 ou R est choisi parmi un radical alkyle ; un radical hydroxyalkyle ; un radical aminoalkyle ; un radical alcoxy ; un radical hydroxyalcoxy. A titre d'exemple, R peut être un radical méthyle ; un radical hydroxyméthyle ; un radical 2-hydroxyéthyle ; un radical 1,2-dihydroxyéthyle ; un radical méthoxy ; un radical 2-hydroxyéthoxy.

Selon un mode de réalisation préféré de l'invention, n est égal à 0 ou 1.

Selon un mode de réalisation particulier de l'invention, R₁ et R₂ sont choisis parmi un atome d'hydrogène ; un radical alkyle ; un radical alkylcarbonyle ; un radical carboxyle ; un radical carbamoyle ; un radical mono ou dialkylcarbamoyle ; un radical hydroxyalkyle. A titre d'exemple, R₁ et R₂ peuvent être un atome d'hydrogène ; un radical hydroxyméthyle ; un radical méthyle ; un radical *n*-butyle.

Selon un mode de réalisation particulier de l'invention, m est égal à 0 ou R₃ est choisi parmi un radical hydroxyle ; un radical alcoxy ; un radical amino ; un radical mono ou dialkylamino ; un radical alkyle ; un radical hydroxyalkyle ; un radical carbamoyle ; un radical mono ou dihydroxyalkylamino ; un radical mésylate ; un radical méthylsulfonamide ; un radical hydroxyalcoxy. A titre d'exemple, R₃ peut être un radical diméthylamino ; un radical hydroxyle ; un radical 2-hydroxyéthylamino ; un radical N,N-bis-(2-hydroxyéthyl)amino ; un radical n-propylamino ; un radical méthyle ; un radical isopropyloxy ; un radical mésylate ; un radical méthylsulfonamide ; un radical amino ; un radical 2-hydroxyéthoxy ; un radical n-butyle ; un radical *tert*-butyle.

Selon un mode de réalisation préféré de l'invention, m est égal à 0, 1 ou 4.

Les dérivés de para-phénylènediamine de formule (I) peuvent être éventuellement salifiés par des acides minéraux forts tels que par exemple HCI, HBr, H₂SO₄, ou des acides organiques tels que, par exemple, l'acide acétique, lactique, tartrique, citrique ou succinique.

A titre d'exemples des dérivés de para-phénylènediamine de formule (I), on peut citer les composés présentés dans le tableau ci-dessous.

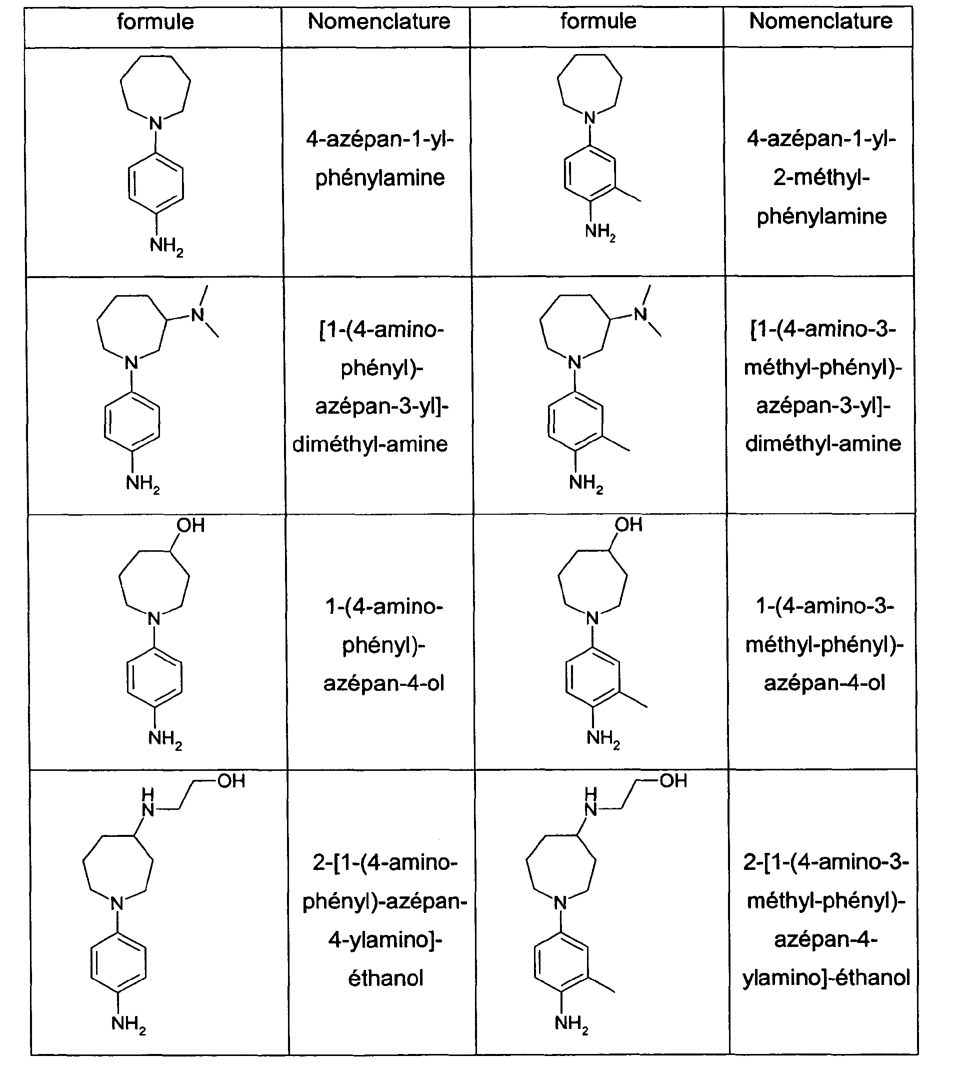

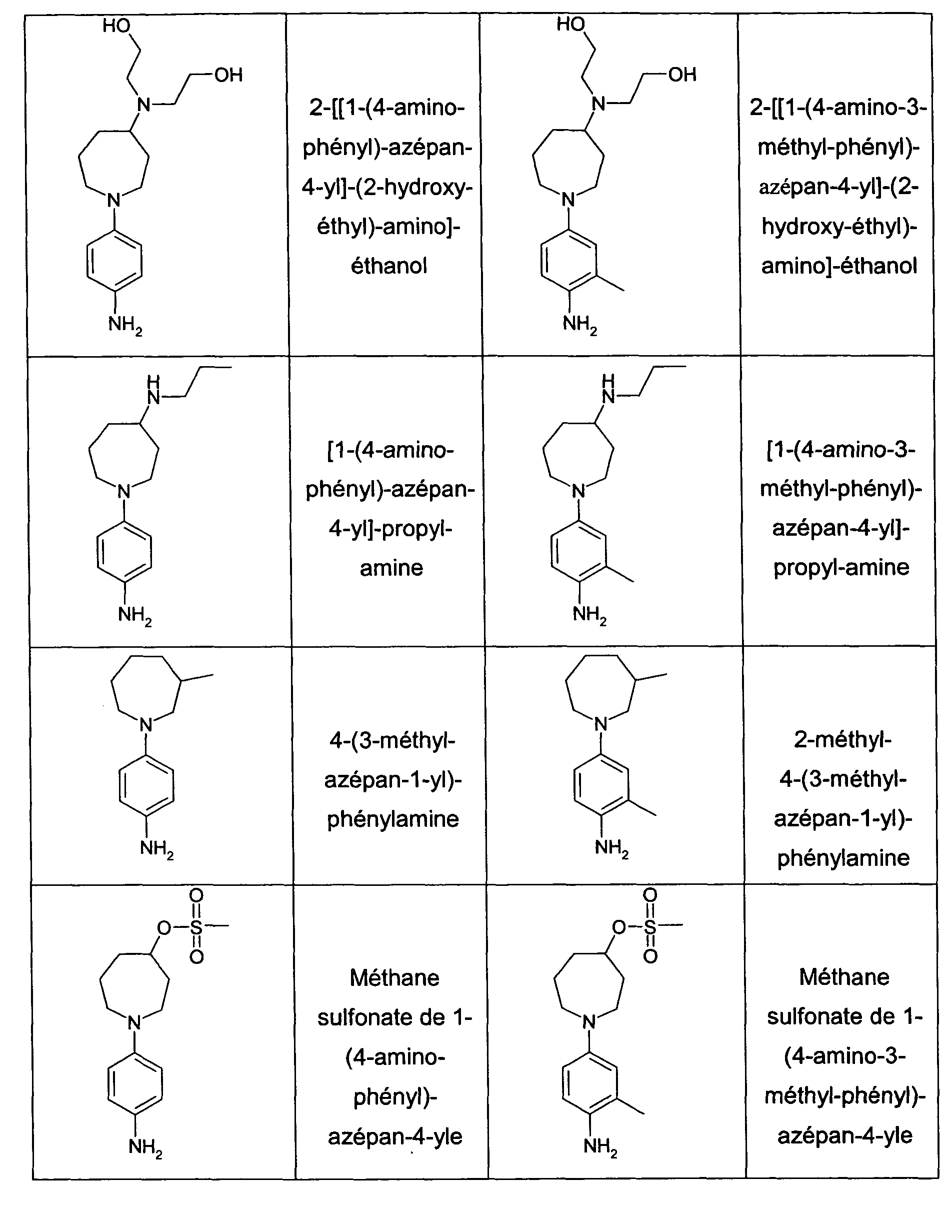

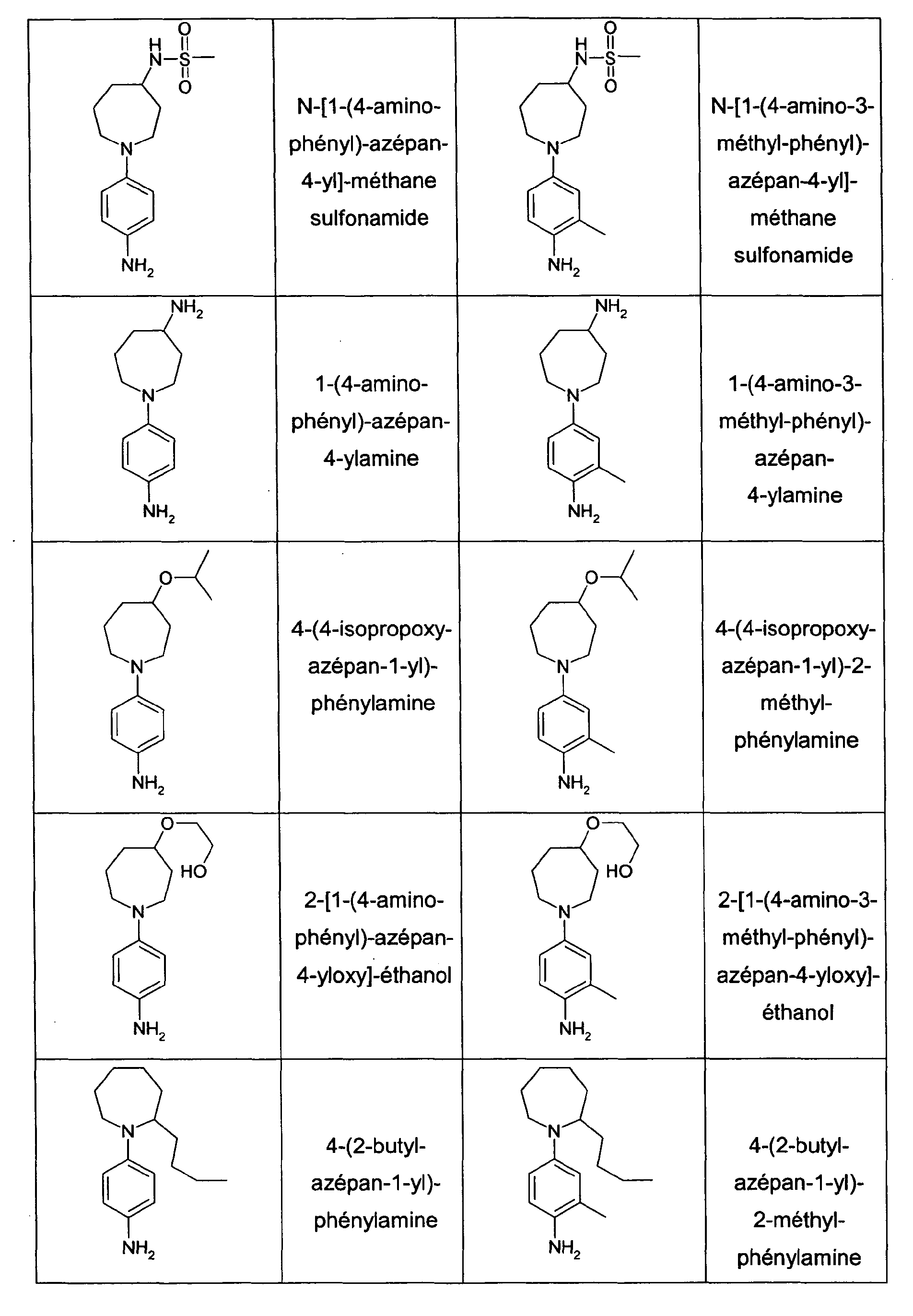

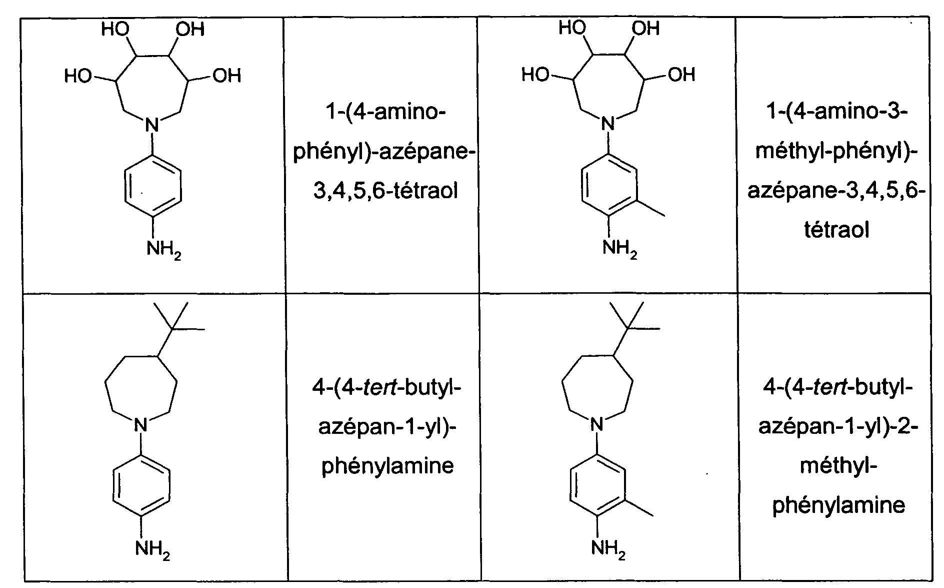

Parmi ces composés, les dérivés de para-phénylènediamine de formule (I) particulièrement préférés sont la 4-azépan-1-yl-phénylamine, la 4-azépan-1-yl-2-méthyl-phénylamine et le 1-(4-amino-phényl)-azépan-4-ol .

Le carbone substitué par R₁, R₂ et R₃ peut être de configuration (R)
ou (S).

Le procédé de la présente invention est un procédé dans lequel une composition tinctoriale comprenant, dans un milieu de teinture approprié, au moins une base d'oxydation choisie parmi les dérivés de para-phénylènediamine de formule (I) et leurs sels d'addition est appliquée sur les fibres kératiniques, et la couleur est révélée à l'aide d'un agent oxydant.

La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition tinctoriale.

Selon un mode de réalisation particulier, la composition comprenant le dérivé de para-phénylènediamine de formule (I) et / ou l'un de ses sels d'addition est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition tinctoriale de l'invention peut contenir un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les para-phénylènediamines autres que celles décrites précédemment, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition tinctoriale sont en général présentes chacune en quantité comprise entre 0,001 à 10 % en poids environ du poids total de la composition tinctoriale, de préférence entre 0,005 et 6 %.

D'une manière générale, les bases d'oxydation et les coupleurs utilisables dans le cadre de l'invention peuvent être éventuellement salifiés par des acides minéraux forts tel que par exemple HCI, HBr, H₂SO₄, ou des acides organiques tels que par exemple, l'acide acétique, lactique, tartrique, citrique ou succinique.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 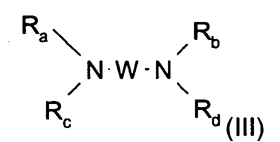 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. II peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une' teinture des fibres kératiniques, et notamment des cheveux humains.

L'invention a aussi pour objet un dispositif à plusieurs compartiments ou "kit" de teinture dans lequel un premier compartiment renferme la composition tinctoriale comprenant au moins une base d'oxydation choisie parmi les dérivés de para-phénylènediamine de formule (I) et leurs sels d'addition et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation choisie parmi les dérivés de para-phénylènediamine de formule (I) et leurs sels d'addition avec un agent oxydant, et l'application du mélange obtenu sur les fibres kératiniques pendant un temps suffisant pour développer la coloration désirée.

Parmi les dérivés de para-phénylènediamine de formule (I) tels que définis précédemment, un certain nombre sont déjà connus en eux-mêmes et décrits dans le brevet DE 42 41 532 : le 1-(4-amino-3-méthyl-phényl)-azépane-4,5-diol et le 1-(4-amino-3-méthyl-phényl)-azépane-3,6-diol et dans le brevet US 2 943 109 : la 4-azépan-1-yl-phénylamine, pour une utilisation en photographie. Le brevet US 2 943 109 décrit également une composition comprenant de la 4-azépan-1-yl-phénylamine dans l'eau et l'éthanol.

Les compositions comprenant, dans un milieu de teinture approprié, au moins une base d'oxydation choisie parmi les dérivés de para-phénylènediamine de formule (I') et leurs sels d'addition : 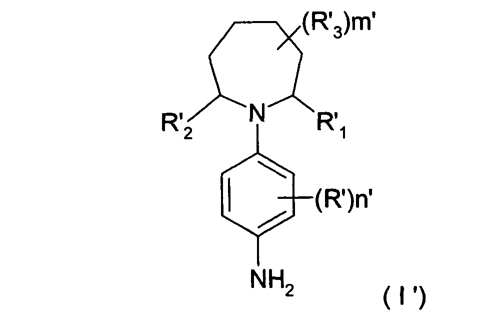 dans laquelle :
- R' représente :
   - un radical alkyle ;
   - un radical hydroxyalkyle ;
   - un radical aminoalkyle ;
   - un radical hydroxy et aminoalkyle ;
   - un radical alcoxy ;
   - un radical alcoxyalkyle ;
   - un radical hydroxyalcoxy ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical carboxyalkyle ;
   - un radical carbamoylalkyle ;
- n' est compris entre 0 et 4, étant entendu que lorsque n' est supérieur à 1 alors les radicaux R' peuvent être identiques ou différents ;
- R'₁ et R'₂ représentent :
   - un atome d'hydrogène ;
   - un radical alkyle saturé ou insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical hydroxyaminoalkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono ou dialkylaminocarbonyle ;
- R'₃ représente :
   - un radical alkyle saturé ou insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical hydroxyaminoalkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono ou dialkylaminocarbonyle ;
   - un radical hydroxyle ;
   - un radical alcoxy ;
   - un radical hydroxyalcoxy ;
   - un radical amino ;
   - un radical mono ou dialkylamino ;
   - un radical mono ou dihydroxyalkylamino ;
   - un radical mésylate ;
   - un radical méthylsulfonamide ;
   - un radical tosylate ;
   - un radical benzènesulfonamide ;
- m' est compris entre 0 et 8, étant entendu que lorsque m' est supérieur à 1 alors les radicaux R'₃ peuvent être identiques ou différents ;
étant entendu que l'un au moins des radicaux R'₁ ou R'₂ est différent d'un atome d'hydrogène ou l'un au moins des entiers n' ou m' est différent de 0 ;
à l'exception du 1-(4-amino-3-méthyl-phényl)-azépane-4,5-diol et ses sels d'addition ;
sont nouvelles et constituent un autre objet de l'invention.

Dans les compositions conformes à l'invention, le ou les dérivés de para-phénylènediamine de formule (I') sont de préférence choisis parmi les dérivés de para-phénylènediamine de formule (I") et leurs sels d'addition : 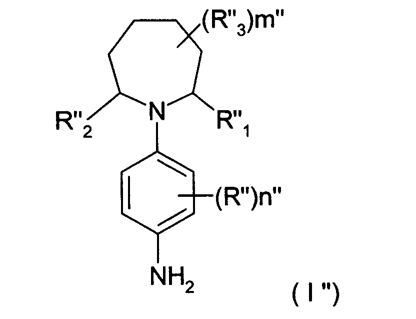 dans laquelle :
- R" représente :
   - un radical alkyle ;
   - un radical hydroxyalkyle ;
   - un radical aminoalkyle ;
   - un radical hydroxy et aminoalkyle ;
   - un radical alcoxy ;
   - un radical alcoxyalkyle ;
   - un radical hydroxyalcoxy ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical carboxyalkyle ;
   - un radical carbamoylalkyle ;
- n" est compris entre 0 et 4, étant entendu que lorsque n" est supérieur à 1 alors les radicaux R" peuvent être identiques ou différents ;
- R"₁ et R"₂ représentent :
   - un atome d'hydrogène ;
   - un radical alkyle saturé ou insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical hydroxyaminoalkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono ou dialkylaminocarbonyle ;
- R"₃ représente :
   - un radical alkyle saturé ou insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical hydroxyaminoalkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono ou dialkylaminocarbonyle ;
   - un radical alcoxy ;
   - un radical hydroxyalcoxy ;
   - un radical amino ;
   - un radical mono ou dialkylamino ;
   - un radical mono ou dihydroxyalkylamino ;
   - un radical mésylate ;
   - un radical méthylsulfonamide ;
   - un radical tosylate ;
   - un radical benzènesulfonamide ;
- m" est compris entre 0 et 8, étant entendu que lorsque m" est supérieur à
   1 alors les radicaux R"₃ peuvent être identiques ou différents ; étant entendu que l'un au moins des radicaux R"₁ ou R"₂ est différent d'un atome d'hydrogène ou l'un au moins des entiers n" ou m" est différent de 0.

Les dérivés de para-phénylènediamine définis ci-dessus autres que le 1-(4-amino-3-méthyl-phényl)-azépane-3,6-diol et ses sels d'addition sont nouveaux et constituent un autre objet de l'invention.

Les dérivés de para-phénylènediamine de formule (I) sont obtenus par réduction des dérivés de para-nitroaniline de formule (II) suivante et leurs sels d'addition : 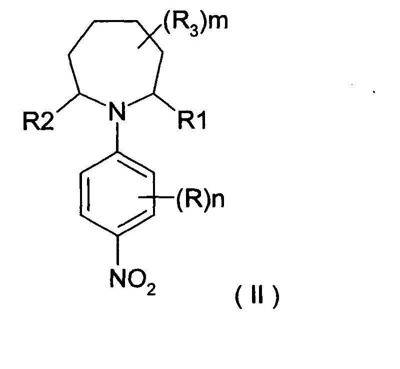 dans laquelle les radicaux R, R₁, R₂ et R₃ et les entiers n et m sont tels que définis précédemment ;
à l'exception du 1-(4-nitro-2,5-diéthoxy-phényl)-azépane, du 1-(4-nitro-phényl)-azépane, du 1-(4-nitro-2-méthyl-phényl)-azépane et leurs sels d'addition.

Parmi les dérivés de para-nitroaniline définis ci-dessus, on préfère les dérivés de para-nitroaniline de formule (II') et leurs sels d'addition : 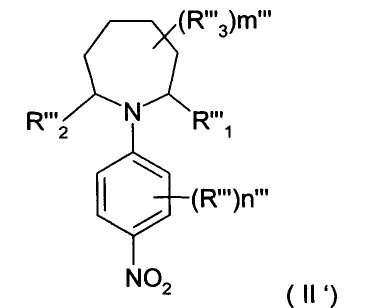 dans laquelle :
- R''' représente :
   - un radical hydroxyalkyle ;
   - un radical aminoalkyle ;
   - un radical hydroxy et aminoalkyle ;
   - un radical alcoxyalkyle ;
   - un radical hydroxyalcoxy ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical carboxyalkyle ;
   - un radical carbamoylalkyle ;
- n''' est compris entre 0 et 4, étant entendu que lorsque n"' est supérieur à 1 alors les radicaux R"' peuvent être identiques ou différents ;
- R'''₁ et R'''₂ représentent :
   - un atome d'hydrogène ;
   - un radical alkyle saturé ou insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical hydroxyaminoalkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono ou dialkylaminocarbonyle ;
- R'''₃ représente :
   - un radical alkyle saturé ou insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine étant non substituée ou mono ou disubstituée par un radical alkyle, acétyle, hydroxyalkyle ;
   - un radical hydroxyaminoalkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono ou dialkylaminocarbonyle ;
   - un radical hydroxyle ;
   - un radical alcoxy ;
   - un radical hydroxyalcoxy ;
   - un radical amino ;
   - un radical mono ou dialkylamino ;
   - un radical mono ou dihydroxyalkylamino ;
   - un radical mésylate ;
   - un radical méthylsulfonamide ;
   - un radical tosylate ;
   - un radical benzènesulfonamide ;
- m"' est compris entre 0 et 8, étant entendu que lorsque m"' est supérieur à 1 alors les radicaux R'''₃ peuvent être identiques ou différents ;
étant entendu que l'un au moins des radicaux R'''₁ ou R'''₂ est différent d'un atome d'hydrogène ou l'un au moins des entiers n"' ou m"' est différent de 0.

Les exemples qui suivent servent à illustrer l'invention sans toutefois présenter un caractère limitatif.

### EXEMPLES

### Exemple 1 : Synthèse du 4-azépan-1-yl-phénylamine

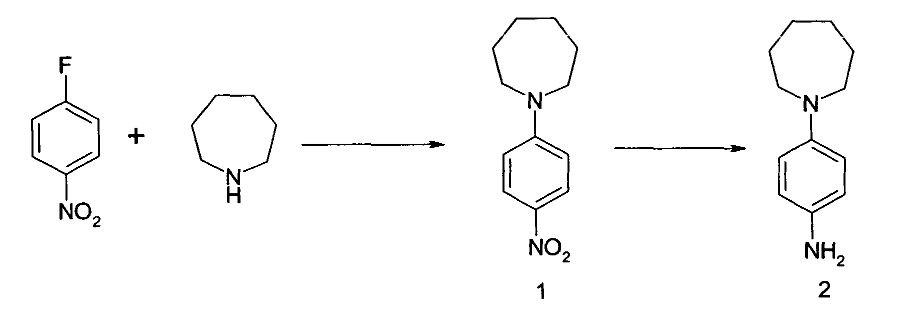

### Etape 1 : Préparation du 1-(4-nitro-phényl)azépane (1)

20 g de para-fluoronitrobenzène (0,141 mole), 16,7 g d'hexaméthylèneimine (0,168 mole) et 48,8 g de carbonate de sodium (0,168 mole) d'eau sont maintenus dans 150 mL à 83 °C sous agitation pendant 4 h 30. Après refroidissement à température ambiante, le solide jaune obtenu est filtré et rincé abondamment à l'eau jusqu'à ce que le pH des eaux de lavage soit neutre. Après séchage, 27 g de produit sont obtenus, soit un rendement de 89 %. Les résultats d'analyse sont les suivants :
¹H RMN (400 MHz, DMSO) : 1,4 (m, 4H) ; 1,6 (m, 4H) ; 3.5 (m, 4H) ; 6,75 (m, 2H) ; 8,0 (m, 2H).
Microanalyse : Théorie (%) : C : 65,44 ; H : 7,32 ; N : 12,72 ; O : 14,53.
Trouvée (%) : C : 64,89 ; H : 7,30 ; N : 12,71 ; O : 14,48.

### Etape 2 : Préparation du 4-azépan-1-yl-phénylamine (2)

Dans un réacteur de 2 L, 25 g de 1-(4-nitro-phényl)azépane (1) obtenu précédemment (0,124 mole) et 5 g de palladium sur charbon à 5% sont placés dans 500 mL de méthanol. L'hydrogénation est réalisée à température ambiante et sous 5 bars de pression d'hydrogène pendant 45 minutes. Après filtration du catalyseur, 29 g de produit sont isolés sous forme de chlorhydrate, soit un rendement de 89 %. Les résultats d'analyse sont les suivants :
¹H RMN (400 MHz, D₂O) : 1,88 ( m, 4H) ; 2,(m, 4H) ; 3,83 (m, 4H) ; 7,67 (m, 2H) ; 7,84 (m, 2H).
Masse ESI+ : m/z = 190 (M+).

### Exemple 2 : Synthèse du 4-azépan-1-yl-2-méthyl-phénylamine

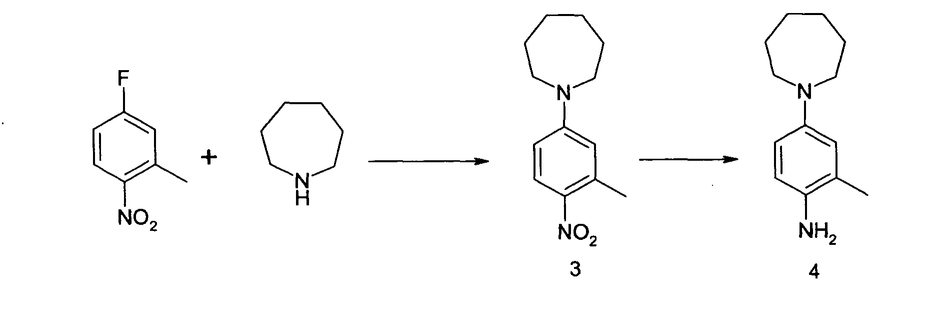

### Etape 1 : Préparation du 1-(3-méthyl-4-nitro-phényl)azépane (3)

32,6 g de 2-méthyl-para-fluoronitrobenzène (0,210 mole), 25 g d'hexaméthyleneimine (0,253 mole) et 35 g de carbonate de potassium (0,253 mole) sont maintenus dans 130 mL d'eau à 95 °C pendant 3 jours. Après refroidissement à température ambiante, le solide marron obtenu est filtré puis recristallisé dans l'éthanol pour donner 40 g d'un solide jaune, soit un rendement de 81 %. Les résultats d'analyse sont les suivants :
¹H RMN (400 MHz, DMSO) : 1,3 (m, 4H) ; 1,5 (m, 4H) ; 2,4 (s, 3H) ; 3,4 (4H); 6,5 (m, 2 H) ; 7,8 (m, 1 H).

### Etape 2 : Préparation du 4-azépan-1-yl-2-méthyl-phénylamine (4)

Dans un hydrogénateur, 15 g de 1-(3-méthyl-4-nitro-phényl)azépane (3) obtenu précédemment (0,064 mole) et 5,4 g de palladium sur charbon à 5 % sont placés dans 650 mL de d'éthanol. L'hydrogénation est réalisée à température ambiante et sous 10 bars de pression d'hydrogène pendant 2 h 30. Après filtration du catalyseur, 13 g de produit sont isolés sous forme de chlorhydrate, soit un rendement de 73 %. Les résultats d'analyse sont les suivants :
¹H RMN (400 MHz, D₂O) : 1,86 (m, 4H) ; 2,1 (m, 4H) ; 2,47 (s, 3H) ; 3,78 (m, 4H) ; 7,57 (d, 1 H) ; 7,6 (dd, 1 H) ; 7,68 (d, 1 H).
Masse ESI+ : m/z = 205 (M+).

### Exempte 3 : Synthèse du 1-(4-amino-phényl)-azépan-4-ol

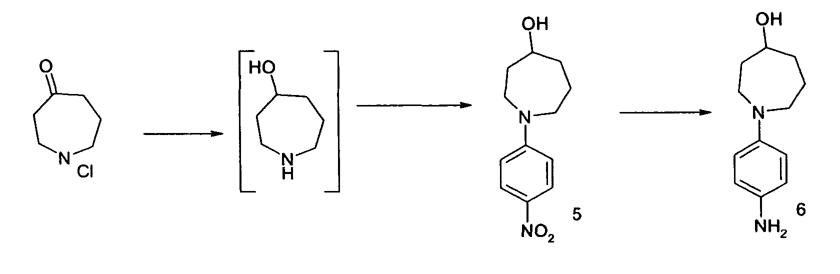

### Etape 1 : Réduction de l'hexahydro-4H-azépine-4-one en azépan-4-ol

Dans un tricol 250 mL, 5 g d'hexahydro-4H-azépine-4-one (0.0334 mole) sont introduits dans 50 mL d'éthanol absolu. A la suspension beige obtenue, 1 g de borohydrure de sodium (0.05 mol) sont ajoutés sous argon et à 10°C par petites fractions. La réaction est laissée 1 heure à température ambiante.

### Etape 2 : Préparation 1-(4-nitro-phenyl)-azépan-4-ol (5)

A la suspension obtenue ci-dessus, 7,1 g de para-fluoronitrobenzène (0.05 mole) sont ajoutés à température ambiante. Le mélange jaune fluorescent obtenu est chauffé 6 heures à reflux. Après évaporation de l'éthanol, une extraction à l'acétate d'éthyle est réalisée. La phase organique obtenue est séchée puis évaporée à sec pour donner 8,5 g d'une poudre jaune. La chromatographie du produit brut sur gel de silice avec l'éluant CH₂CI₂ / MeOH (95 / 5) conduit à 5,9 g d'un solide jaune vif, soit un rendement de 75 %.
¹H RMN (400 MHz, DMSO) : 1,53-1,68 (m, 4H) ; 1,92 (m, 2H) ; 3,45-3,58 (m, 4H) ; 3,71 (m, 1 H) ; 4,56 (d , 1 H) ; 6,78 (m, 2H) ; 8,02 (m, 2H).
Masse (ESI+/- dans MeOH / H2O) : m / z = 237 (MH)⁺ ; 235 (MH)⁻ ; 259 (MNa)⁺.

### Etape 3 : Préparation du 1-(4-amino-phényl)-azépan-4-ol hydrochloride (6)

Dans un hydrogénateur, 4 g de 1-(4-nitro-phényl)-azépan-4-ol (0,017 mole) et 0,8 g de palladium sur charbon à 5% sont placés dans 150 mL de d'éthanol. L'hydrogénation est réalisée à température ambiante et sous 10 bars de pression d'hydrogène pendant 2 h 30. Après filtration du catalyseur, 4,3 g de produit sont isolés sous forme de chlorhydrate, soit un rendement de 91%. Les résultats d'analyse sont les suivants :
¹H RMN (400 MHz, D₂O) : 1,91 (m, 1 H) ; 2,05 (m, 1H) ; 2,23 (m, 3H) ; 2,43 (m, 1 H) ; 3,81 (m, 3H) ; 3,96 (m, 1 H) ; 4,25 (m, 1 H) ; 7,67 (m, 2H) ;7,82 (m, 2H).
Masse ESI+ : 272 (MH)+.

### EXEMPLES DE TEINTURE

### Exemples 1 à 6 : composition tinctoriale à partir 4-azépan-1-yl-phénylamine

### -Exemples 1 à 2 : teinture en milieu acide

Les compositions tinctoriales suivantes sont préparées :

| **Exemple** | **1** | **2** |
|---|---|---|
| 4-azépan-1-yl-phénylamine | 4.10⁻⁴ mole | 4.10⁻⁴ mole |
| 2-(2,4-diamino-phénoxy)-éthanol ; chlorhydrate | 4.10⁻⁴ mole | - |
| 3-amino 2-chloro 6-méthyl phénol ; chlorhydrate | - | 4.10⁻⁴ mole |
| Support de teinture (1) | (*) | (*) |
| Eau déminéralisée q.s.p. | 100 g | 100 g |

| | | |
|---|---|---|
| (*) : support de teinture (1 ) pH 7 | | |

| | |
|---|---|
| Alcool éthylique à 96° | 20,8 g |
| Métabisulfite de sodium en solution aqueuse à 35% Sel pentasodique de l'acide diéthylène-triamine-pentaacétique | 0,23 g M.A |
| en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| **Exemple** | **1** | **2** |
|---|---|---|
| **Nuance observée** | Bleu | Bleu |

### -Exemples 3 à 6 : teinture en milieu basique

Les compositions tinctoriales suivantes sont préparées :

| **Exemple** | **3** | **4** | **5** | **6** |
|---|---|---|---|---|
| 4-azépan-1-yl-phénylamine | 4.10⁻⁴ mole | 4.10⁻⁴ mole | 4.10⁻⁴ mole | 4.10⁻⁴ mole |
| 5-amino 2-méthyl phénol | 4.10⁻⁴ mole | - | - | - |
| 2-(2,4-diamino-phénoxy)-éthanol ; chlorhydrate | - | 4.10⁻⁴ mole | - | - |
| 3,6-diméthyl-1H-pyrazolo[5,1-c] [1,2,4]triazole | - | - | 4.10⁻⁴ mole | - |
| 3-amino 2-chloro 6-méthyl phénol ; chlorhydrate | - | - | - | 4.10⁻⁴ mole |
| Support de teinture (2) | (*) | (*) | (*) | (*) |
| Eau déminéralisée q.s.p | 100 g | 100 g | 100 g | 100 g |

| | | | | |
|---|---|---|---|---|
| (*) : support de teinture (2) pH 9,5 | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| **Exemple** | **3** | **4** | **5** | **6** |
|---|---|---|---|---|
| **Nuance observée** | Bleu violet | Bleu | Violet rouge | Bleu |

### Exemples 7 à 17 : composition tinctoriale à partir du 4-azépan-1-yl-2-méthyl-phénylamine

### - Exemples 7 à 11 : teinture en milieu acide

Les compositions tinctoriales suivantes sont préparées :

| **Exemple** | **7** | **8** | **9** | **10** | **11** |
|---|---|---|---|---|---|
| 4-azépan-1-yl-2-méthyl-phénylamine | 4.10⁻⁴ mole | 4.10⁻⁴ mole | 4.10⁻⁴ mole | 4.10⁻⁴ mole | 4.10⁻⁴ mole |
| 5-amino 2-méthyl phénol | 4.10⁻⁴ mole | - | - | - | - |
| 2-(2,4-diamino-phénoxy)-éthanol ; chlorhydrate | - | 4.10⁻⁴ mole | - | - | - |
| 2-amino pyridin-3-ol - | | - | 4.10⁻⁴ mole - | | - |
| 1H-indol-6-ol | - | - | - | 4.10⁻⁴ mole | - |
| 3-amino 2-chloro 6-méthyl phénol ; chlorhydrate | - | - | - | - | 4.10⁻⁴ mole |
| Support de teinture (1) | (*) | (*) | (*) | (*) | (*) |
| Eau déminéralisée q.s.p. | 100 g | 100 g | 100 g | 100 g | 100 g |

| | | | | | |
|---|---|---|---|---|---|
| (*) : support de teinture (1) pH 7 | | | | | |

| | |
|---|---|
| Alcool éthylique à 96° | 20,8 g |
| Métabisulfite de sodium en solution aqueuse à 35% Sel pentasodique de l'acide diéthylène-triamine-pentaacétique | 0,23 g M.A |
| en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| **Exemple** | **7** | **8** | **9** | **10** | **11** |
|---|---|---|---|---|---|
| **Nuance observée** | Bleu | Bleu vert | Violet | Violet | Bleu |

### -Exemples 12 à 16 : teinture en milieu basique

Les compositions tinctoriales suivantes sont préparées :

| **Exemple** | **12** | **13** | **14** | **15** | **16** |
|---|---|---|---|---|---|
| 4-azépan-1-yl-2-méthyl-phénylamine | 4.10⁻⁴ mole | 4.10⁻⁴ mole | 4.10⁻⁴ mole | 4.10⁻⁴ mole | 4.10⁻⁴ mole |
| 5-amino 2-méthyl phénol | 4.10⁻⁴ mole | - | - | - | - |
| 2-(2,4-diamino-phénoxy)-éthanol ; chlorhydrate | - | 4.10⁻⁴ mole | - | - | - |
| 2-amino pyridin-3-ol - | | - | 4.10⁻⁴ mole - | | - |
| 1H-indol-6-ol | - | - | - | 4.10⁻⁴ mole | - |
| 3,6-diméthyl-1 H-pyrazolo-[5,1-c] [1,2,4]triazole | - | - | - | - | 4.10⁻⁴ mole |
| Support de teinture (2) | (*) | (*) | (*) | (*) | (*) |
| Eau déminéralisée q.s.p. | 100 g | 100 g | 100 g | 100 g | 100 g |

| | | | | | |
|---|---|---|---|---|---|
| (*) : support de teinture (2) pH 9,5 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄CI | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| **Exemple** | **12** | **13** | **14** | **15** | **16** |
|---|---|---|---|---|---|
| **Nuance observée** | Bleu | Bleu | Violet | Violet | Violet rouge |

### Exemples 17 à 30 : composition tinctoriale à partir 1-(4-aminophényl)azépan-4-ol

### -Exemples 17 à 23 : teinture en milieu acide

Les compositions tinctoriales suivantes sont préparées :

| **Exemple** | **17** | **18** | **19** | **20** | **21** | **22** | **23** |
|---|---|---|---|---|---|---|---|
| 1-(4-aminophényl) azépan-4-ol | 4.10⁻⁴ mole | 4.10⁻⁴ mole | 4.10⁻⁴ mole | 4.10⁻⁴ mole | 4.10⁻⁴ mole | 4.10⁻⁴ mole | 4.10⁻⁴ mole |
| Benzène-1,3-diol | 4.10⁻⁴ mole | - | - | - | - | - | - |
| 5-amino 2-méthyl -phénol | | 4.10⁻⁴ mole | - | - | - | - | - |
| 1H-indol-6-ol | - | - | 4.10⁻⁴ mole | - | - | - | - |
| 2-amino pyridin-3-ol | - | - | - | 4.10⁻⁴ mole | - | - | - |
| 3,6-diméthyl-1H-pyrazolo[5,1-c] [1,2,4]triazole | - | - | - | - | 4.10⁻⁴ mole | - | - |
| 2-(2,4-diamino-phénoxy)-éthanol ; chlorhydrate | - | - | - | - | - | 4.10⁻⁴ mole | - |
| 3-amino 2-chloro 6-méthyl phénol ; chlorhydrate | - | - | - | - | - | - | 4.10⁻⁴ mole |
| Support de teinture (2) | (*) | (*) | (*) | (*) | (*) | (*) | (*) |
| Eau déminéralisée q.s.p. | 100 g | 100 g | 100 g | 100 g | 100 g | 100 g | 100 g |

| | | | | | | | |
|---|---|---|---|---|---|---|---|
| (*) : support de teinture (1 ) pH 7 | | | | | | | |

| | |
|---|---|
| Alcool éthylique à 96° | 20,8 g |
| Métabisulfite de sodium en solution aqueuse à 35% Sel pentasodique de l'acide diéthylène-triamine-pentaacétique | 0,23 g M.A . |
| en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableauci-dessous:

| **Exemple** | **17** | **18** | **19** | **20** | **21** | **22** | **23** |
|---|---|---|---|---|---|---|---|
| **Nuance observée** | Brun | Violet bleu intense | Gris violet intense | Gris violet intense | Violet intense | Bleu intense | Bleu intense |

### -Exemples 24 à 30 : teinture en milieu acide Basique

Les compositions tinctoriales suivantes sont préparées :

| **Exemple** | **24** | **25** | **26** | **27** | **28** | **29** | **30** |
|---|---|---|---|---|---|---|---|
| 1-(4-aminophényl) azépan-4-ol | 4.10⁻⁴ mole | 4.10⁻⁴ mole | 4.10⁻⁴ mole | 4.10⁻⁴ mole | 4.10⁻⁴ mole | 4.10⁻⁴ mole | 4.10⁻⁴ mole |
| Benzène-1,3-diol | 4.10⁻⁴ mole | - | - | - | - | - | - |
| 5-amino 2-méthyl-phénol | - | 4.10⁻⁴ mole | - | - | - | - | - |
| 1H-indol-6-ol | - | - | 4.10⁻⁴ mole | - | - | - | - |
| 2-amino pyridin-3-ol | - | - | - | 4.10⁻⁴ mole | - | - | - |
| 3,6-diméthyl-1 H-pyrazolo[5,1-c] [1,2,4]triazole | - | - | - | - | 4.10⁻⁴ mole | - | - |
| 2-(2,4-diamino-phénoxy)-éthanol ; chlorhydrate | - | - | - | - | - | 4.10⁻⁴ mole | - |
| 3-amino 2-chloro 6-méthyl phénol ; chlorhydrate | - | - | - | - | - | - | 4.10⁻⁴ mole |
| Support de teinture (2) | (*) | (*) | (*) | (*) | (*) | (*) | (*) |
| Eau déminéralisée q.s.p. | 100 g | 100 g | 100 g | 100 g | 100 g | 100 g | 100 g |

| | | | | | | | |
|---|---|---|---|---|---|---|---|
| (*) : support de teinture (1) pH 9,5 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄CI | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableauci-dessous:

| **Exemple** | **24** | **25** | **26** | **27** | **28** | **29** | **30** |
|---|---|---|---|---|---|---|---|
| **Nuance observée** | Brun | Violet bleu intense | Brun intense | Gris violet intense | Violet intense chromatique | Bleu intense | Bleu intense |

## Revendications

1. Utilisation, pour la teinture des fibres kératiniques, d'un dérivé de para-phénylènediamine substituée par un noyau homopipéridine de formule (I) et / ou de l'un de ses sels d'addition : 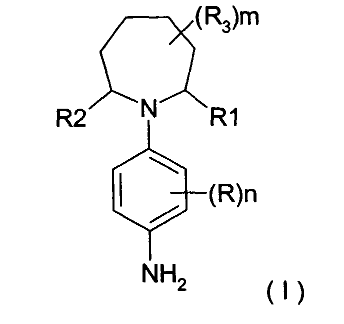 dans laquelle :
• R représente :
- un radical alkyle ;
- un radical hydroxyalkyle ;
- un radical aminoalkyle ;
- un radical hydroxy et aminoalkyle ;
- un radical alcoxy ;
- un radical alcoxyalkyle ;
- un radical hydroxyalcoxy ;
- un radical hydroxyalcoxyalkyle ;
- un radical aminoalkyle, l'amine étant non substituée ou mono ou disubstituée par un radical alkyle, acétyle, hydroxyalkyle ;
- un radical carboxyalkyle ;
- un radical carbamoylalkyle ;
• n est compris entre 0 et 4, étant entendu que lorsque n est supérieur à 1 alors les radicaux R peuvent être identiques ou différents ;
• R₁ et R₂ représentent :
- un atome d'hydrogène ;
- un radical alkyle saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine étant non substituée ou mono ou disubstituée par un radical alkyle, acétyle, hydroxyalkyle ;
- un radical hydroxyaminoalkyle ;
- un radical carboxyle ;
- un radical carboxyalkyle ;
- un radical carbamoyle ;
- un radical carbamoylalkyle ;
- un radical alcoxycarbonyle ;
- un radical mono ou dialkylaminocarbonyle ;
• R₃ représente :
- un radical alkyle saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine étant non substituée ou mono ou disubstituée par un radical alkyle, acétyle, hydroxyalkyle ;
- un radical hydroxyaminoalkyle ;
- un radical carboxyle ;
- un radical carboxyalkyle ;
- un radical carbamoyle ;
- un radical carbamoylalkyle ;
- un radical alcoxycarbonyle ;
- un radical mono ou dialkylaminocarbonyle ;
- un radical hydroxyle ;
- un radical alcoxy ;
- un radical hydroxyalcoxy ;
- un radical amino ;
- un radical mono ou dialkylamino ;
- un radical mono ou dihydroxyalkylamino ;
- un radical mésylate ;
- un radical méthylsulfonamide ;
- un radical tosylate ;
- un radical benzènesulfonamide ;
• m est compris entre 0 et 8, étant entendu que lorsque m est supérieur à 1 alors les radicaux R₃ peuvent être identiques ou différents.

2. Utilisation selon la revendication 1 dans laquelle n est égal à 0 ou R est choisi parmi un radical alkyle ; un radical hydroxyalkyle ; un radical aminoalkyle ; un radical alcoxy ; un radical hydroxyalcoxy.

3. Utilisation selon la revendication 2 dans laquelle n est égal à 0 ou R est choisi parmi un radical méthyle ; un radical hydroxyméthyle ; un radical 2-hydroxyéthyle ; un radical 1,2-dihydroxyéthyle ; un radical méthoxy ; un radical 2-hyd roxyéthoxy.

4. Utilisation selon l'une quelconque des revendications précédentes dans laquelle n est égal à 0 ou 1.

5. Utilisation selon l'une quelconque des revendications précédentes dans laquelle R₁ et R₂ sont choisis parmi un atome d'hydrogène ; un radical alkyle ; un radical alkylcarbonyle ; un radical carboxyle ; un radical carbamoyle ; un radical mono ou dialkylcarbamoyle ; un radical hydroxyalkyle.

6. Utilisation selon la revendication 5 dans laquelle R₁ et R₂ sont choisis parmi un atome d'hydrogène ; un radical hydroxyméthyle ; un radical méthyle ; un radical n-butyle.

7. Utilisation selon l'une quelconque des revendications précédentes dans laquelle m est égal à 0 ou R₃ est choisi parmi un radical hydroxyle ; un radical alcoxy ; un radical amino ; un radical mono ou dialkylamino ; un radical alkyle ; un radical hydroxyalkyle ; un radical carbamoyle ; un radical mono ou dihydroxyalkylamino ; un radical mésylate ; un radical méthylsulfonamide ; un radical hydroxyalcoxy.

8. Utilisation selon la revendication 7 dans laquelle m est égal à 0 ou R₃ est choisi parmi un radical diméthylamino ; un radical hydroxyle ; un radical 2-hydroxyéthylamino ; un radical N,N-bis-(2-hydroxyéthyl)amino ; un radical *n*-propylamino ; un radical méthyle ; un radical isopropyloxy ; un radical mésylate ; un radical méthylsulfonamide ; un radical amino ; un radical 2-hydroxyéthoxy ; un radical *n*-butyle ; un radical *tert*-butyle.

9. Utilisation selon l'une quelconque des revendications précédentes dans laquelle m est égal à 0, 1 ou 4.

10. Utilisation selon l'une quelconque des revendications précédentes dans laquelle le dérivé de para-phénylènediamine de formule (I) est choisi parmi la 4-azépan-1-yl-phénylamine ; la 4-azépan-1-yl-2-méthyl-phénylamine ; la [1-(4-amino-phényl)-azépan-3-yl]-diméthyl-amine ; la [1-(4-amino-3-méthyl-phényl)-azépan-3-yl]-diméthyl-amine ; le 1-(4-amino-phényl)-azépan-4-ol ; le 1-(4-amino-3-méthyl-phényl)-azépan-4-ol ; le 2-[1-(4-amino-phényl)-azépan-4-yl-amino]-éthanol ; le 2-[1-(4-amino-3-méthyl-phényl)-azépan-4-yl-amino]-éthanol ; le 2-[[1-(4-amino-phényl)-azépan-4-yl]-(2-hydroxy-éthyl)-amino]-éthanol ; le 2-[[1-(4-amino-3-méthyl-phényl)-azépan-4-yl]-(2-hydroxy-éthyl)-amino]-éthanol ; la [1-(4-amino-phényl)-azépan-4-yl]-propyl-amine ; la [1-(4-amino-3-méthyl-phényl)-azépan-4-yl]-propyl-amine ; la 4-(3-méthyl-azépan-1-yl)-phényl-amine ; la 2-méthyl-4-(3-méthyl-azépan-1-yl)-phénylamine ; le méthanesulfonate de 1-(4-amino-phényl)-azépan-4-yle ; le méthanesulfonate de 1-(4-amino-3-méthyl-phényl)-azépan-4-yle ; le N-[1-(4-amino-phényl)-azépan-4-yl]-méthanesulfonamide ; le N-[1-(4-amino-3-méthyl-phényl)-azépan-4-yl]-méthanesulfonamide ; la 1-(4-amino-phényl)-azépan-4-yl-amine ; la 1-(4-amino-3-méthyl-phényl)-azépan-4-yl-amine ; la 4-(4-isopropoxy-azépan-1-yl)-phénylamine ; la 4-(4-isopropoxy-azépan-1-yl)-2-méthyl-phénylamine ; le 2-[1-(4-amino-phényl)-azépan-4-yloxy]-éthanol ; le 2-[1-(4-amino-3-méthyl-phényl)-azépan-4-yloxy]-éthanol ; la 4-(2-butyl-azépan-1-yl)-phénylamine ; la 4-(2-butyl-azépan-1-yl)-2-méthyl-phénylamine ; le 1-(4-amino-phényl)-azépane-3,4,5,6-tétraol ; le 1-(4-amino-3-méthyl-phényl)-azépane-3,4,5,6-tétraol ; la 4-(4-*tert*-butyl-azépan-1-yl)-phénylamine ; la 4-(4-*tert*-butyl-azépan-1-yl)-2-méthyl-phénylamine.

11. Utilisation selon la revendication 10 dans laquelle le dérivé de para-phénylènediamine de formule (I) est choisi parmi la 4-azépan-1-yl-phénylamine ; la 4-azépan-1-yl-2-méthyl-phénylamine et le 1-(4-amino-phényl)-azépan-4-ol.

12. Procédé de teinture des fibres kératiniques, **caractérisé en ce qu'**une composition tinctoriale comprenant, dans un milieu de teinture approprié, au moins une base d'oxydation choisie parmi les dérivés de para-phénylènediamine substituée par un noyau homopipéridine de formule (I) tels que définis dans l'une quelconque des revendications 1 à 11 et leurs sels d'addition est appliquée sur les fibres kératiniques en présence d'un agent oxydant pendant un temps suffisant pour développer la coloration désirée.

13. Procédé selon la revendication 12 dans lequel la composition tinctoriale comprend de plus un ou plusieurs coupleurs choisis parmi les méta-phénylènediamines, les méta-aminophénols, les méta-diphénols, les coupleurs naphtaléniques, les coupleurs hétérocycliques et leurs sels d'addition.

14. Procédé selon la revendication 13 dans lequel la quantité de chacun des coupleurs est comprise entre 0,001 et 10 % en poids du poids total de la composition tinctoriale.

15. Procédé selon l'une quelconque des revendications 12 à 14 dans lequel la composition tinctoriale comprend une ou plusieurs bases d'oxydation additionnelle choisie parmi les para-phénylènediamines autres que les dérivés de para-phénylènediamine de formule (I), les bis-phénylalkylènediamines, les para-aminophénols, les bis-para-aminophénols, les ortho-aminophénols, les bases hétérocycliques et leurs sels d'addition.

16. Procédé selon l'une quelconque des revendications 12 à 15 dans lequel la quantité de chacune des bases d'oxydation est comprise entre 0,001 et 10 % en poids du poids total de la composition tinctoriale.

17. Procédé selon l'une quelconque des revendications 12 à 16 dans lequel l'agent oxydant est choisi parmi le peroxyde d'hydrogène, le peroxyde d'urée, les bromates de métaux alcalins, les persels, les peracides et les enzymes oxydases.

18. Dispositif à plusieurs compartiments dans lequel un premier compartiment contient une composition tinctoriale telle que définie dans l'une quelconque des revendications 12 à 16 et un deuxième compartiment contient un agent oxydant.

19. Composition tinctoriale des fibres kératiniques comprenant, dans un milieu de teinture approprié, au moins une base d'oxydation choisie parmi les dérivés de para-phénylènediamine de formule (I') et leurs sels d'addition : 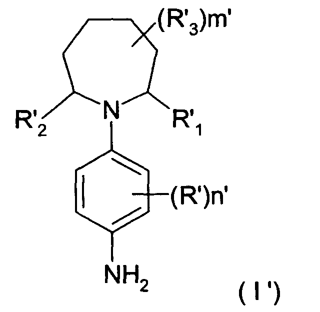 dans laquelle :
• R' représente :
- un radical alkyle ;
- un radical hydroxyalkyle ;
- un radical aminoalkyle ;
- un radical hydroxy et aminoalkyle ;
- un radical alcoxy ;
- un radical alcoxyalkyle ;
- un radical hydroxyalcoxy ;
- un radical hydroxyalcoxyalkyle ;
- un radical aminoalkyle, l'amine étant non substituée ou mono ou disubstituée par un radical alkyle, acétyle, hydroxyalkyle ;
- un radical carboxyalkyle ;
- un radical carbamoylalkyle ;
• n'est compris entre 0 et 4, étant entendu que lorsque n' est supérieur à 1 alors les radicaux R' peuvent être identiques ou différents ;
• R'₁ et R'₂ représentent :
- un atome d'hydrogène ;
- un radical alkyle saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine étant non substituée ou mono ou disubstituée par un radical alkyle, acétyle, hydroxyalkyle ;
- un radical hydroxyaminoalkyle ;
- un radical carboxyle ;
- un radical carboxyalkyle ;
- un radical carbamoyle ;
- un radical carbamoylalkyle ;
- un radical alcoxycarbonyle ;
- un radical mono ou dialkylaminocarbonyle ;
• R'₃ représente :
- un radical alkyle saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine étant non substituée ou mono ou disubstituée par un radical alkyle, acétyle, hydroxyalkyle ;
- un radical hydroxyaminoalkyle ;
- un radical carboxyle ;
- un radical carboxyalkyle ;
- un radical carbamoyle ;
- un radical carbamoylalkyle ;
- un radical alcoxycarbonyle ;
- un radical mono ou dialkylaminocarbonyle ;
- un radical hydroxyle ;
- un radical alcoxy ;
- un radical hydroxyalcoxy ;
- un radical amino ;
- un radical mono ou dialkylamino ;
- un radical mono ou dihydroxyalkylamino ;
- un radical mésylate ;
- un radical méthylsulfonamide ;
- un radical tosylate ;
- un radical benzènesulfonamide ;
• m' est compris entre 0 et 8, étant entendu que lorsque m' est supérieur à 1 alors les radicaux R'₃ peuvent être identiques ou différents ;
étant entendu que l'un au moins des radicaux R'₁ ou R'₂ est différent d'un atome d'hydrogène ou l'un au moins des entiers n' ou m' est différent de 0 ; à l'exception du 1-(4-amino-3-méthyl-phényl)-azépane-4,5-diol et ses sels d'addition.

20. Composition selon la revendication 19, dans laquelle le ou les dérivés de para-phénylènediamine de formule (I') sont choisis parmi les dérivés de para-phénylènediamine de formule (I") et leurs sels d'addition : 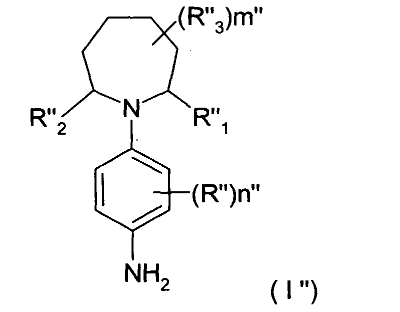 dans laquelle :
• R" représente :
- un radical alkyle ;
- un radical hydroxyalkyle ;
- un radical aminoalkyle ;
- un radical hydroxy et aminoalkyle ;
- un radical alcoxy ;
- un radical alcoxyalkyle ;
- un radical hydroxyalcoxy ;
- un radical hydroxyalcoxyalkyle ;
- un radical aminoalkyle, l'amine étant non substituée ou mono ou disubstituée par un radical alkyle, acétyle, hydroxyalkyle ;
- un radical carboxyalkyle ;
- un radical carbamoylalkyle ;
• n" est compris entre 0 et 4, étant entendu que lorsque n" est supérieur à 1 alors les radicaux R" peuvent être identiques ou différents ;
• R"₁ et R"₂ représentent :
- un atome d'hydrogène ;
- un radical alkyle saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine étant non substituée ou mono ou disubstituée par un radical alkyle, acétyle, hydroxyalkyle ;
- un radical hydroxyaminoalkyle ;
- un radical carboxyle ;
- un radical carboxyalkyle ;
- un radical carbamoyle ;
- un radical carbamoylalkyle ;
- un radical alcoxycarbonyle ;
- un radical mono ou dialkylaminocarbonyle ;
• R"₃ représente :
- un radical alkyle saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine étant non substituée ou mono ou disubstituée par un radical alkyle, acétyle, hydroxyalkyle ;
- un radical hydroxyaminoalkyle ;
- un radical carboxyle ;
- un radical carboxyalkyle ;
- un radical carbamoyle ;
- un radical carbamoylalkyle ;
- un radical alcoxycarbonyle ;
- un radical mono ou dialkylaminocarbonyle ;
- un radical alcoxy ;
- un radical hydroxyalcoxy ;
- un radical amino ;
- un radical mono ou dialkylamino ;
- un radical mono ou dihydroxyalkylamino ;
- un radical mésylate ;
- un radical méthylsulfonamide ;
- un radical tosylate ;
- un radical benzènesulfonamide ;
• m" est compris entre 0 et 8, étant entendu que lorsque m" est supérieur à
1 alors les radicaux R"₃ peuvent être identiques ou différents ; étant entendu que l'un au moins des radicaux R"₁ ou R"₂ est différent d'un atome d'hydrogène ou l'un au moins des entiers n" ou m" est différent de 0.

21. Dérivés de para-phénylènediamine tels que définis dans la revendication 19 ou 20 à l'exclusion du 1-(4-amino-3-méthyl-phényl)-azépane-3,6-diol et ses sels d'addition.

22. Dérivés de para-nitroaniline de formule (II) suivante et leurs sels d'addition : 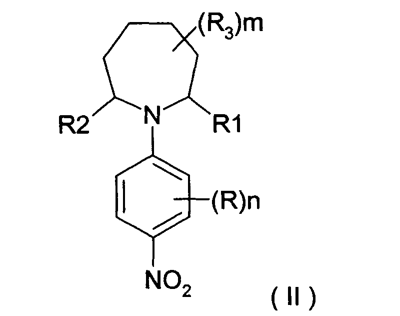 dans laquelle les radicaux R, R₁, R₂ et R₃ et les entiers n et m sont tels que définis dans l'une quelconque des revendications 1 à 11 ; à l'exception du 1-(4-nitro-2,5-diéthoxy-phényl)-azépane, du 1-(4-nitro-phényl)-azépane, du 1-(4-nitro-2-méthyl-phényl)-azépane et leurs sels d'addition.

23. Dérivés selon la revendication 22 choisis parmi les dérivés de para-nitroaniline de formule (II') et leurs sels d'addition : 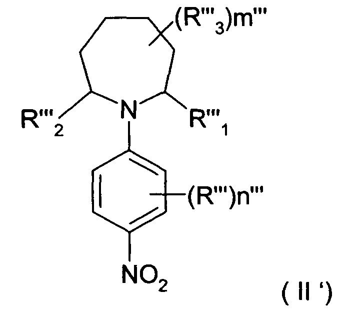 dans laquelle :
• R''' représente :
- un radical hydroxyalkyle ;
- un radical aminoalkyle ;
- un radical hydroxy et aminoalkyle ;
- un radical alcoxyalkyle ;
- un radical hydroxyalcoxy ;
- un radical hydroxyalcoxyalkyle ;
- un radical aminoalkyle, l'amine étant non substituée ou mono ou disubstituée par un radical alkyle, acétyle, hydroxyalkyle ;
- un radical carboxyalkyle ;
- un radical carbamoylalkyle ;
• n''' est compris entre 0 et 4, étant entendu que lorsque n"' est supérieur à 1 alors les radicaux R"' peuvent être identiques ou différents ;
• R'''₁ et R'''₂ représentent :
- un atome d'hydrogène ;
- un radical alkyle saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine étant non substituée ou mono ou disubstituée par un radical alkyle, acétyle, hydroxyalkyle ;
- un radical hydroxyaminoalkyle ;
- un radical carboxyle ;
- un radical carboxyalkyle ;
- un radical carbamoyle ;
- un radical carbamoylalkyle ;
- un radical alcoxycarbonyle ;
- un radical mono ou dialkylaminocarbonyle ;
• R'''₃ représente :
- un radical alkyle saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine étant non substituée ou mono ou disubstituée par un radical alkyle, acétyle, hydroxyalkyle ;
- un radical hydroxyaminoalkyle ;
- un radical carboxyle ;
- un radical carboxyalkyle ;
- un radical carbamoyle ;
- un radical carbamoylalkyle ;
- un radical alcoxycarbonyle ;
- un radical mono ou dialkylaminocarbonyle ;
- un radical hydroxyle ;
- un radical alcoxy ;
- un radical hydroxyalcoxy ;
- un radical amino ;
- un radical mono ou dialkylamino ;
- un radical mono ou dihydroxyalkylamino ;
- un radical mésylate ;
- un radical méthylsulfonamide ;
- un radical tosylate ;
- un radical benzènesulfonamide ;
• m"' est compris entre 0 et 8, étant entendu que lorsque m''' est supérieur
à 1 alors les radicaux R"'₃ peuvent être identiques ou différents ; étant entendu que l'un au moins des radicaux R'''₁ ou R'''₂ est différent d'un atome d'hydrogène ou l'un au moins des entiers n"' ou m"' est différent de 0.
